# EUROPÄISCHE PATENTANMELDUNG

(11) **EP 0 688 570 A2**
(43) Veröffentlichungstag der Anmeldung: **27.12.1995**
(21) Anmeldenummer: 95107383.2
(22) Anmeldetag: 16.05.1995
(51) Int. Cl.: A61M 5/178, A61M 5/24

(54) **Insulinspritze**

(30) Priorität: 21.06.1994 DE 4421617; 03.02.1995 DE 19503474
(71) Anmelder: Gmeiner, Wilhelm, D-92224 Amberg (DE)
(72) Erfinder: Gmeiner, Wilhelm, D-92224 Amberg (DE); Gmeiner, Karin, D-92224 Amberg (DE)
(74) Vertreter: Wasmeier, Alfons, Dipl.-Ing.

(57) **Zusammenfassung**

Bei einer Insulinspritze mit auswechselbarer Injektionsnadel und mit der Nadel verbindbarer auswechselbarer Insulinampulle besteht die Nadel aus zwei voneinander beabstandeten, im wesentlichen koaxialen Nadelteilen. Die beiden Nadelteile sind durch eine Durchflußsperrvorrichtung voneinander getrennt. Der äußere Nadelteil, nämlich die Injektionsnadel, ist in einem die Nadel aufnehmenden Körper des Nadelgehäuses befestigt, und der innere Nadelteil, nämlich die Verbindungsnadel zur Insulinampulle, ist in einem im Nadelgehäuse festgelegten Führungskörper angeordnet. Die Durchflußsperrvorrichtung ist vorzugsweise eine flexible Membrane mit einer sich selbst schließenden Durchtrittsstelle, die so wirkt, daß unter Druckeinwirkung Insulinflüssigkeit aus der Insulinampulle von den einen in den anderen Nadelteil fließen kann, und daß ohne Druckeinwirkung die elastische Durchtrittsstelle der Membrane den Durchfluß zum äußeren Nadelteil sperrt. Die Membrane ist insbesondere ein Plättchen oder eine Folie aus Gummi oder ähnlich elastisch nachgiebigem Material.

## Beschreibung

Die Erfindung betrifft eine Insulinspritze nach dem Oberbegriff des Anspruches 1.

Die erfolgreiche Behandlung bzw. Beherrschung und Therapie der Diabetes hängt entscheidend davon ab, daß der Patient eine Insulinspritze zur Verfügung hat, mit deren Hilfe er seinen Insulinbedarf selbst injizieren kann. Hierfür ist entscheidend, daß mit der Spritze auf möglichst einfache und exakte Weise aus dem das Insulin enthaltenden Behälter die gewünschte Dosis Insulin in den Körper injiziert werden kann.

Den derzeit auf dem Markt befindlichen unterschiedlichen, im Prinzip jedoch ähnlich arbeitenden Insulinspritzen ist gemeinsam, daß über einen von Hand betätigbaren Kolben das in einem Behälter enthaltene Insulin in eine Nadel gedrückt und über das Einstechende der Nadel in den Körper des Patienten injiziert wird. Allen bekannten Spritzen dieser Art ist jedoch gemeinsam, daß sie eine durchgehende Nadel aufweisen und daß damit eine ständige Verbindung zwischen dem Insulin im Behälter und der Außenluft über die Nadel besteht. Dadurch tropft häufig ohne Zutun des Benutzers Insulin aus der Ampulle aus und Luft strömt in die Ampulle ein. Wird nach dem Injizieren der Fingerdruck vom Nachschubkolben genommen, entspannt sich der Gummikolben in der Ampulle kaum merkbar nach hinten, wodurch Luft durch die Injektionsnadel in die Ampulle angesaugt wird. Desweiteren tritt durch Austropfen oder Verdunsten von Insulin Luft in die Ampulle ein. Diese in die Insulinampulle eingedrungene Luft muß vom Patienten vor jeder Injektion unbedingt entfernt werden, damit sichergestellt ist, daß aufgrund der eingedrungenen Luft Fehldosierungen beim Spritzen von Insulin sicher vermieden werden. Dieses Entfernern der Luft aus der Insulinampulle ist mühsam und nimmt Zeit in Anspruch. Außerdem kommen ältere Personen mit der Handhabung der Spritze zum Entlüften nur schwierig oder überhaupt nicht zurecht.

Es ist ferner häufig der Fall, daß beim Injizieren Blutgefäße angestochen werden. Das dabei austretende Blut wird nach dem Injiziervorgang beim Wegnehmen des Druckes von der Kolbenstange und durch den sich dabei leicht nach hinten entspannenden Gummikolben in der Insulinampulle durch die durchgehende Nadel hindurch in die Ampulle gesaugt, wodurch das wertvolle Insulin unbrauchbar wird.

Aufgabe der Erfindung ist es, die vorhandenen, gattungsgemäßen Insulinspritzen auf besonders einfache und effektive Weise so zu verbessern, daß ein selbständiges unerwünschtes Austreten oder Verdunsten von Insulinflüssigkeit aus der Ampulle und somit ein Eindringen von Luft durch die Injektionsnadel oder ein Ansaugen von Blut in die Ampulle sicher vermieden wird und bei jeder Injektion das Entlüften entfällt und die eingestellte Insulinmenge jederzeit dosisgenau injiziert werden kann.

Gemäß der Erfindung wird diese Aufgabe mit den Merkmalen des Kennzeichens des Anspurches 1 erreicht. Weitere Ausgestaltungen der Erfindung sind Gegenstand der Unteransprüche.

Während bei der Verwendung einer durchgehenden Nadel eine ständige Verbindung zwischen dem Insulin in der Ampulle bzw. in einer Kartusche, in einem Fläschchen oder dergl., und der freien Luft am Nadeleinstechende besteht, ist im Falle vorliegender Erfindung diese Verbindung über eine mit einer im Ruhezustand verschlossenen Durchtrittsstelle versehene Membrane oder eine entsprechende Ventilvorrichtung unterbunden, so daß keine Luft bzw. kein Blut in die Insulinampulle gelangen kann.

Da bei der Vorrichtung nach der Erfindung im Ruhezustand der Spritze der Insulinfluß von der Insulinampulle zum freien Ende der Spitze der Injektionsnadel unterbrochen ist, kann dabei auch ohne Kolbenbetätigung, z. B. durch Erschütterung, kein Insulin durch die Nadel austreten und keine Luft in die Ampulle einströmen. Damit ist gewährleistet, daß die Spritze jederzeit zur genau dosierten und sofortigen Abgabe der vom Patienten gewünschten, eingestellten Insulindosis bereit ist und der Patient sicher sein kann, daß er kein Insulin-Luft-Gemisch spritzt.

Die elastisch nachgiebige Membrane, die eine im wesentlichen zentrische, elastische, sich selbst schließende Durchtrittsstelle (oder mehrere Durchtrittsstellen mit gleichem oder unterschiedlichem Querschnitt, falls eine erhöhte Fördermenge bzw. Insulingabe erfolgt, z.B., wenn eine große Anzahl von Insulineinheiten auf einmal verabreicht werden soll, der Kolben mit mehr Kraft und/oder stoßartig betätigt wird, oder dergl.) aufweist, ist so beschaffen, daß bei Ausübung eines Druckes auf den Kolben Insulin aus der Ampulle und dem inneren Nadelteil durch die elastische Durchtrittsstelle bzw. Durchtrittsstellen hindurchtritt und in den äußeren Nadelabschnitt hineinströmt und durch die äußere Nadel in den Körper gespritzt werden kann. Wird die Druckwirkung aufgehoben, wird aufgrund der Elastizität des Materials durch Zusammenziehen der Membrane diese Durchtrittsstelle verschlossen und der Insulindurchfluß gesperrt, so daß kein Blut und keine Luft durch die Nadel in die Insulinampulle gelangen kann. Die Membrane ist zweckmäßigerweise in einem Volumen zwischen den beiden einander zugewandten Flächen des äußeren und des inneren Führungskörpers, die jeweils einen der beiden Nadelabschnitte aufnehmen, angeordnet und weist eine Wulst, insbesondere eine Ringwulst auf, die im äußeren und/oder im inneren Körper einen Sitz hat, so daß das Volumen innerhalb der Wulst durch die einander zugewandten Flächen der beiden Führungskörper begrenzt und abgedichtet ist und die Membrane einen festen Sitz hat. Im Ruhezustand ist das Volumen der Membrane, das der Ampulle zugewandt ist, mit Insulin gefüllt, das durch die im Ruhezustand geschlossene, im wesentlichen zentrische Durchtrittsstelle in der Membrane nicht abfließen kann. Wird zur Injektion Druck auf den Kolben und damit auf das Insulinvolumen in der Ampulle ausgeübt, fließt Insulin aufgrund des ausgeübten Druckes durch die Durchtrittsstelle und von dort durch die Injektionsnadel in den menschlichen Körper.

Die Nadelanordnung nach der Erfindung besteht aus dem Nadelgehäuse mit einem die Injektionsnadel aufnehmenden Körper, der fest mit dem Gehäuse verbunden ist, dem die Insulinentnahmenadel aufnehmenden Körper, der in das Gehäuse dem Injektionsnadelkörper zugeordnet eingesetzt ist, der als Membrane ausgebildeten Sperrvorrichtung zwischen den beiden Körpern, und einem Spritzengehäuse mit darin auswechselbar aufgenommener Insulinampulle. Diese Ampulle, ein Glasfläschchen oder eine Kartusche, weist am Abgabeende einen Verschluß auf, der von der Spitze der Insulinentnahmenadel durchstochen wird, wenn das Spritzengehäuse mit Insulinampulle mit dem Nadelbehälter verbunden, z.B. verschraubt wird. Beim Verbinden des Spritzengehäuses mit dem Nadelgehäuse durchdringt die dem Gehäuse zugewandte Insulinentnahmenadel den Verschluß der Insulinampulle, so daß Insulin in die Nadel und durch die Nadel gedrückt werden kann. Der die Insulinentnahmenadel aufnehmende Körper wird in das Nadelgehäuse eingesetzt und dort so festgelegt, z.B. durch Einschrauben, Einrasten, Einkleben, Einschweißen oder dergl., daß er mit dem Nadelkörper in Anlage steht und beide miteinander ein Volumen ausbilden, das die Sperrvorrichtung, z.B. eine Membrane aufnimmt, wobei die Sperrvorrichtung vor dem Einsetzen des Insulinentnahmenadelkörpers eingesetzt wird. Die beiden Körper können wahlweise vertauscht sein, so daß der Einsetzkörper der äußere Körper ist.

Bei einer weiteren Ausgestaltung der Erfindung wird vorgeschlagen, die beiden den Nadelkörper bildenden Teile an den einander zugewandten Flächen mit Erhebungen und Vertiefungen auszugestalten, die im zusammengebauten Zustand ineinandergreifend ausgebildet sind, und eine Membrane in Form einer elastisch nachgiebigen Folie zwischen die beiden Körperteile zu legen, so daß die Erhebungen im einen Körperteil die Folie in die entsprechenden Gegenvertiefungen des anderen Körperteiles drücken und damit die Folie festlegen, wodurch eine Abdichtung erreicht wird.

Bei einer weiter abgeänderten Ausführungsform der Erfindung werden in den beiden die Nadelkörper darstellenden Körperteilen keine Erhebungen bzw. Vertiefungen vorgesehen, sondern die beiden einander zugewandten Flächen sind im wesentlichen plan. Zwischen beide Flächen wird eine Membrane in Form einer elastisch nachgiebigen Folie eingelegt und diese Folie wird an einer der beiden einander zugewandten Flächen z.B. durch Kleben oder durch mechanisches Einklemmen unter gleichzeitiger Abdichtung festgelegt, so daß der Druck, der durch das eine Körperteil auf das andere Körperteil des Nadelkörpers ausgeübt wird, die Festlegung der Folie ergibt. Bei dieser Ausführungsform können die beiden einander zugewandten Flächen der Körperteile des Nadelkörpers gerauht, geriffelt oder dergl. ausgebildet sein, um ein Festlegen der Folie zu verbessern, wenn ein Verkleben der Folie nicht erwünscht ist. Auch bei diesen zusätzlichen Ausführungsformen ist die Folie mit einer elastischen Durchtrittstelle versehen, die unter Druckeinwirkung steht, damit im belasteten Zustand der Durchtritt von Insulin sichergestellt ist, im unbelasteten Zustand jedoch ein Durchtritt von Insulin und ein Eindringen von Luft oder ein Ansaugen von Blut von außen verhindert wird.

Die Bezeichnung "Durchtrittsstelle" bedeutet eine Durchgangsstelle (oder mehrere Durchgangsstellen) in der Membrane, die so bemessen ist, daß sie bei einer Druckbeaufschlagung des Kolbens einen Durchfluß ermöglicht und Insulin aus der Ampulle beim Injizieren abgegeben wird, bei Entlastung des Kolbens jedoch schließt und den Insulindurchfluß auf Grund der Eigenelastizität des Materials der Membrane sperrt. Diese Durchtrittsstelle kann eine Öffnung sein, die z.B. beim Durchstechen der Membrane mit Hilfe einer Nadel entsteht, oder aber ein kleiner, gerader oder gekrümmter Schlitz, der unter Druckeinwirkung sich genügend weit öffnet, um Insulin hindurchtreten zu lassen, bei der Druckentlastung oder nach dem Durchfluß von Insulin durch die Eigenelastizität der Membrane jedoch sich schließt und den Durchfluß sperrt. Die Größe der Durchtrittsstelle(n) hängt dabei maßgeblich sowohl von dem ausgeübten Druck als insbesondere von der Elastizität des Materials ab, also von den Rückstelleigenschaften des Materials, um die Durchflußstelle(n) bei Druckentlastung sicher zu sperren. Wenn beispielsweise die Membrane eine Latexfolie ist, ist es für den einwandfreien Betrieb ausreichend, eine winzige bzw. kleine Öffnung von Nadelspitzenstärke bzw. Nadelstärke vorzusehen.

Anstatt eine Durchflußstelle (oder mehrere Durchflußstellen) und damit eine kleine Öffnung (oder mehrere kleine Öffnungen) in die Membrane, z.B. mit Hilfe einer Nadel (oder mehrer Nadeln) zu stechen, kann das Material für die Membrane auch bereits bei oder nach der Herstellung des Materials so ausgebildet werden, daß es an bestimmten Stellen, die z.B. mit geringerer Dicke ausgeführt sind, durchlässig ist oder wird, wenn Druck auf das Material ausgeübt wird. Durch diese Stellen, die den vorstehend beschriebenen Durchtrittsstellen entsprechen, kann das Material hindurchdiffundieren.

Nachstehend wird die Erfindung in Verbindung mit der Zeichnung anhand eines Ausführungsbeispieles erläutert.
- Fig. 1: zeigt eine erfindungsgemäße Insulinspritze, wobei Nadelgehäuse und Spritzgehäuse voneinander getrennt dargestellt sind,
- Fig. 2: den Nadelkörper in Explosionsdarstellung,
- Fig. 3: eine weitere Ausführungsform der Erfindung, im Schnitt,
- Fig. 4: eine abgeänderte Ausführungsform der Erfindung, im Schnitt,
- Fig. 5: eine andere Ausführungsform der Erfindung in Abänderung der Fig. 1,
- Fig. 6: die Ausführungsform nach Fig. 5 in Explosionsdarstellung,
- Fig. 7: eine gegenüber der Ausführungsform nach Fig. 5 abgeänderte Ausführung, und
- Fig. 8: eine Variante der Ausführungsform nach Fig. 3.

Das Spritzengehäuse 1, vorzugsweise aus Metall oder Kunststoff, nimmt einen Insulinbehälter 2, z.B. eine Insulinampulle aus Glas, die der Form des Gehäuses 1 angepaßt ist, auf. Anstelle einer Glasampulle 2 kann eine Insulinkartusche, z. B. aus Kunststoff, ein Glasfläschchen oder dergl. verwendet werden. Das Spritzengehäuse 1 besitzt einen Hals 3 mit Außengewinde 4 und Öffnung 5, die durch einen den Abschluß der Ampulle 2 bildenden Verschluß 6, z.B. eine dünne Gummischeibe, eine Kunstoffolie oder dergl. verschlossen ist.

Auf das Schraubgewinde 4 des Spritzengehäuses 1 wird das Injektionsnadelgehäuse 7 über das Gewinde 4' aufgeschraubt, das den den Nadelteil 10 aufnehmenden Körper 9 aufweist. Die Nadel 10, 12 besteht aus einem die Injektionsnadel 10 mit Einstechspitze 11 aufweisenden äußeren Teil und einem die Insulinentnahmenadel 12 mit Spitze 13 aufweisenden inneren Teil; letztere ist in einem Einsetzkörper 14 befestigt, der mit dem Spritzengehäuse 7 im eingesetzten Zustand verklebt, verrastet, verschraubt oder in anderer Weise festgelegt ist. Die Körper 9 und 14 bilden mit den einander zugewandten Flächen einen freien Raum, in den die inneren Enden der beiden Nadelteile 10, 12 münden und in dem Vertiefungen 9', 14' ausgebildet sind, die zur Aufnahme einer Sperrvorrichtung 15 dienen. Die Sperrvorrichtung 15 ist beispielsweise als dünne, elastisch nachgiebige Membrane 16 mit Ringwulst 17, vorzugsweise aus Gummi, ausgebildet. Die Kreisfläche der Membrane 16 weist eine im wesentlichen zentrische Durchflußstelle 18 auf, die unter Druckeinwirkung steht, derart, daß mittels eines Kolbens 19 ein Druck in Pfeilrichtung 20 auf das Insulin in der Ampulle 2 ausgeübt wird, der sich durch den Nadelteil 12 gegen die Membrane 15 fortsetzt und Insulin durch die Durchtrittsstelle 18 durchtreten läßt, während in unbelastetem Zustand, also im Ruhestand der Membrane, d.h. wenn kein Druck ausgeübt wird, aufgrund der Elastizität des Materials die Durchtrittsstelle sich schließt und dadurch keine Luft eintreten und kein Insulin aus der Spritze ausfließen kann.

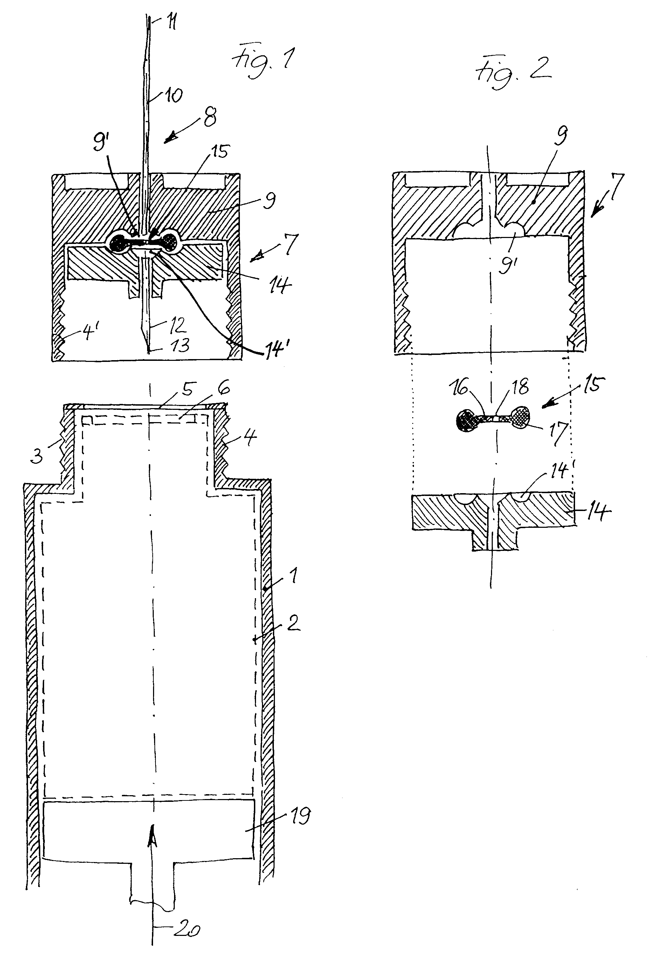

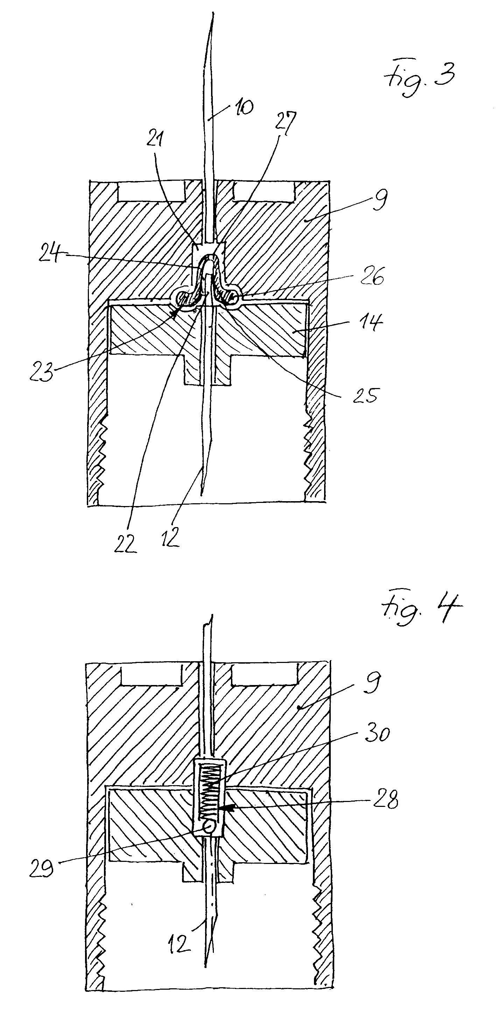

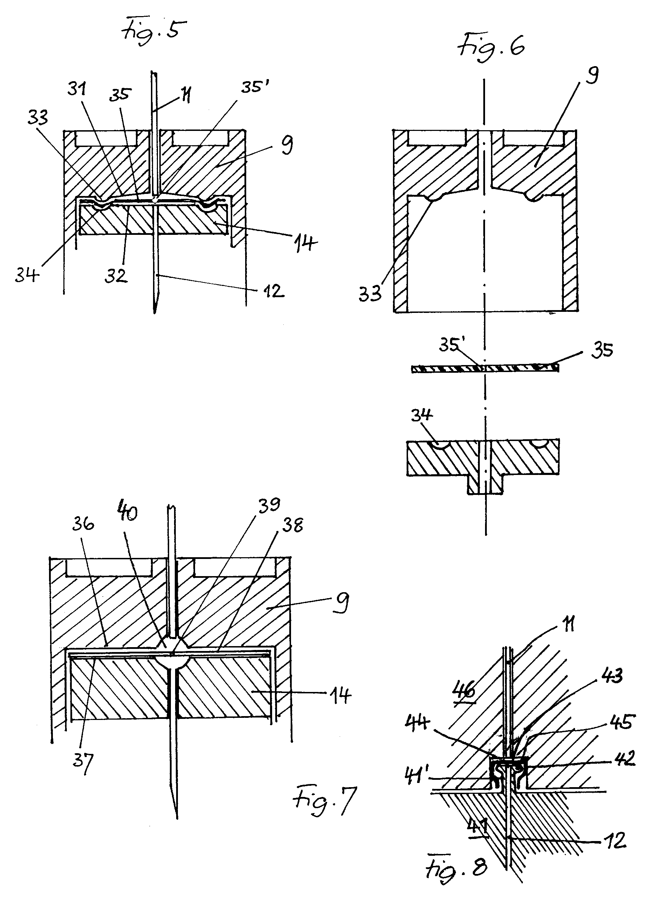

Bei der Ausführungsform der Erfindung nach Figur 3 ist im Körper 9 oder zwischen den beiden Körpern 9 und 14 ein Volumen 21 ausgebildet, in das das innere Ende 22 des Nadelteils 12 vorsteht. Über den vorstehenden Teil 22 ist eine schlauchartige Membrane 23 gestülpt, deren kappenförmiger Teil 24 das innere Nadelende 22 umspannt. Dieser Kappenteil 24 geht radial nach außen in den scheibenförmigen Membranabschnitt 25 und am radial äußeren Rand in die Ringwulst 26 über. Der Membranabschnitt 25 ist zweckmäßigerweise eine Kreisscheibe mit kleinem Querschnitt, und die Ringwulst 26 ist in geringem Abstand von dem Kappenteil 24 ausgebildet, damit ein einwandfreier Sitz des Kappenteiles 24 sichergestellt ist. Der kappenförmige Membranteil 24 hat direkt der inneren Öffnung des Nadelteiles 12 zugewandt eine oder mehrere Durchtrittsstellen, die z.B. mittels Nadeln erzeugt werden, durch die bei Druckeinwirkung beim Injizieren Insulin in den Raum 21 und von dort in den Nadelabschnitt 10 fließen kann. Anstelle einer mit Ringwulst versehenen Membrane kann die Membrane auch ohne Wulst ausgebildet sein, wenn die Membrane anderweitig zwischen den beiden Körpern festgehalten wird.

Anstelle einer Durchtrittsstelle 18 bzw. 27 in einer Membrane, die im Ruhezustand durch die flexible Beschaffenheit des Materials geschlossen ist und erst unter Druckeinwirkung Insulin hindurchfließen läßt, kann in weiterer Ausgestaltung der Erfindung auch ein kleines Ventil, z.B. ein Kugelventil 28 vorgesehen sein, das aus einer Kugel 29 und einer Druckfeder 30 besteht, die die Kugel unter Druckeinwirkung entgegen der Wirkung der Feder von dem inneren Ende des Nadelteils 12 abhebt und damit öffnet. Wird der Druck auf den Kolben weggenommen, druckt die Feder 30 die Kugel 29 gegen ihren Sitz an der Nadel 12 und verhindert ein Auslaufen von Insulin, ein Eintreten von Blut (wenn ein Blutgefäß angestochen wird) oder von Luft in die Insulinampulle. Eine derartige Ausführungsform ist jedoch aufwendig in Hinblick auf Kosten und Platzbedarf, so daß dies keine bevorzugte Lösung darstellt.

Dadurch, daß die Nadel in zwei voneinander durch die Membrane getrennte Abschnitte unterteilt ist, kann Insulin im Ruhezustand nicht von der Innennadel zur Außennadel fließen, da die Öffnung der Membrane bzw. das Ventil im Ruhezustand geschlossen ist. Andererseits kann dadurch auch nicht Luft von außen durch die Nadel in die Insulinampulle gelangen und kein Insulin verdunsten.

Bei der Ausführung nach Fig. 5 sind die einander zugewandten Flächen 31, 32 der Körper 9 und 14 so ausgebildet, daß die Fläche 31 eine umlaufend ringförmige, zusammenhängende Erhebung 33 aufweist, und daß in der Fläche 32 eine entsprechend kongruente bzw. konforme Vertiefung 34 ausgebildet ist, die eine entsprechende, ebenfalls umlaufend ringförmige, zusammenhängende Vertiefung bildet, so daß die Erhebungen 33 in die Vertiefungen 34 passend ausgebildet sind. Diese Vertiefungen und Erhebungen können auch am äußersten Rand des Nadelkörpers 14 ausgebildet sein. Natürlich können die Erhebungen 33 und die Vertiefungen 34 vertauscht vorgesehen sein, so daß dann die Fläche 31 mit Vertiefungen und die Fläche 32 mit Erhebungen ausgebildet ist. Zwischen den beiden Flächen 31, 32 ist eine elastisch nachgiebige, abdichtende Folie aus Kunststoff, Gummi oder dergl. 35 mit Durchtrittsöffnung 35' angeordnet, die sich den Erhebungen und Vertiefungen anpaßt, wenn der Körper 14 gegen den Körper 9 gedrückt wird, und dadurch die Folie über den gesamten Umfang festgehalten wird. Die anstelle der Membrane 15 mit Wulst nach den Figuren 1 - 4 ausgebildete Sperrvorrichtung 35 nach Fig. 5 bzw. Fig. 6 in Form einer Folie stellt eine besonders einfache und zweckmäßige Ausführungsform dar.

Bei der Ausführungsform nach Fig. 7 ist die Anordnung so gewählt, daß die einander zugewandten Flächen 36, 37 der Körper 9, 14 im wesentlichen plan ausgeführt sind, und daß zwischen den Flächen 36, 37 eine Membran 38 in Form einer Folie eingesetzt ist, die somit an den beiden weitgehend planen Flächen 36, 37 in zusammengebautem Zustand des Nadelkörpers eingeklemmt ist. Bei den Ausführungsformen nach den Figuren 5 und 7 ist, wie in den vorausgehenden Figuren, die Membran mit einer (oder mehreren) im wesentlichen zentrischen Durchtrittsstelle 39 versehen, die der zentrischen Durchtrittsstelle 18 nach Fig. 2 entspricht. Die Ausführung nach Fig. 7 ist eine extrem einfache Ausführungsform. Hierbei kann für die Erzielung einer einwandfreien Arbeitsweise die Membran an einer der Flächen 36, 37 festgeklebt werden, um eine einwandfreie Positionierung der Membran zu erzielen und ein Verrutschen zu vermeiden. Mit 40 sind beidseitig der Membran im Nadelbereich vorgesehene Ausnehmungen bezeichnet. Die Membrane 38 ist eben gehalten bzw. leicht gespannt, damit ein stärkeres Durchbiegen der Membrane beim Insulindurchtritt vermieden wird. Dies gilt analog auch für die vorausgehend beschriebenen Ausführungsformen.

Die in Fig. 8 schematisch dargestellte Variante der Ausführungsform nach Fig. 3 sieht vor, daß ein die Nadel 12 aufnehmender Ansatz 41' nur wenig aus dem Körper 41 nach oben vorsteht, daß der vorstehende Teil 41' eine ringförmig umlaufende Erhebung 42, z.B. eine Ringwulst am oberen Ende oder an der zylindrischen Umfangswand aufweist, daß über diese Erhebung 42 eine Membrane bzw. Folie 43 - z.B. ein kurzes Schlauchstück, etwa in Form einer Kappe aus Gummi, Kunststoff oder dergl. Material, das am oberen Ende geschlossen ist und bereits bei der Herstellung mittels einer Nadel oder dergl. für den Durchtritt des Insulins durchstochen wird - gespannt ist, die die Öfffnung 44 der Nadel verschließt, und daß der den vorstehenden Teil 41' der Nadel 12 und die Membrane 43 aufnehmende freie Raum 45 vom Körper 46 umschlossen wird, vorzugsweise so, daß die Membrane 43 durch den Innenrand des Körpers 46 gegen die ringförmige Erhebung 42 in Anlage kommt und damit der Innenraum 45 an den Seiten der Membrane einwandfrei abgedichtet wird.

## Patentansprüche

1. Insulinspritze mit auswechselbarer Injektionsnadel und mit der Nadel verbindbarer auswechselbarer Insulinampulle, die im Spritzengehäuse aufgenommen ist, **dadurch gekennzeichnet**, daß die Nadel aus zwei voneinander beabstandeten, im wesentlichen koaxialen Nadelteilen (10, 12) besteht, daß die beiden Nadelteile (10, 12) durch eine Durchflußsperrvorrichtung (15) voneinander getrennt sind, daß der äußere Nadelteil (10), die Injektionsnadel, in einem die Nadel aufnehmenden Körper (9) des Nadelgehäuses (7) befestigt ist, und daß der innere Nadelteil (12), die Verbindungsnadel zur Insulinampulle (2), in einem im Nadelgehäuse (7) festgelegten Führungskörper (14) angeordnet ist.

2. Vorrichtung nach Anspruch 1, dadurch gekennzeichnet, daß die Durchflußsperrvorrichtung (15) eine flexible Membrane (16) mit im wesentlichen zentrischer, elastischer, sich selbst schließender Durchtrittsstelle (18) ist, und daß die Membrane (16) aus elastisch nachgiebigem Material besteht, derart, daß die Durchtrittsstelle (18) unter Druckeinwirkung (20) Insulinflüssigkeit aus der Insulinampulle (2) durch den Nadelteil (12) in den Nadelteil (10) durchfließen läßt, ohne Druckeinwirkung (20) die elastische Durchtrittsstelle (18) der Membrane (16) den Durchfluß zum Nadelteil (10) jedoch sperrt.

3. Vorrichtung nach Anspruch 1 oder 2, dadurch gekennzeichnet, daß die Membrane (16) aus einem Plättchen aus Gummi oder ähnlichem elastisch nachgiebigem Material und einer äußeren umlaufenden Wulst (17) besteht.

4. Vorrichtung nach Anspruch 3, dadurch gekennzeichnet, daß die Wulst (17) in Vertiefungen (9', 14') des Körpers (9) und/oder des Körpers (14) aufgenommen ist und eine Abdichtung zwischen Körper (9) und Körper (14) darstellt.

5. Vorrichtung nach einem der Ansprüche 1 - 4, dadurch gekennzeichnet, daß der Körper (14) mit dem Nadelteil (12) in das Gehäuse (7) einsetzbar, z.B. einschraubbar, einklebbar, einrastbar oder dergl. ist.

6. Vorrichtung nach einem der Ansprüche 1 - 5, dadurch gekennzeichnet, daß das Gehäuse (7) ein Innengewinde (4') zur Aufnahme eines Außengewindes (3) des Spritzengehäuses (1) aufweist, in dem die Ampulle (2) aufgenommen ist.

7. Vorrichtung nach Anspruch 6, dadurch gekennzeichnet, daß das Spritzengehäuse (1) auf der der Nadel (12) zugewandten Seite eine Öffnung (5) zum Einführen der Nadel (12) und die Ampulle (2) einen durchstechbaren Verschluß (6), der von der Nadel (12) beim Aufschrauben auf das Spritzengehäuse in bekannter Weise durchstochen wird, aufweist.

8. Vorrichtung nach einem der Ansprüche 1 - 7, dadurch gekennzeichnet, daß die Körper (9, 14) auf ihren einander zugewandten Flächen mittige, z.B. konische Ausnehmungen aufweisen, derart, daß die einander zugewandten Enden der Nadelteile (10, 12) in Kammern (9', 14') beidseitig der eben gehaltenen Membrane (16) münden (Fig. 1).

9. Vorrichtung nach Anspruch 1 und 8, dadurch gekennzeichnet, daß die Nadel (12) im Körper (14) in Richtung der Injektionsnadel (10) über die Begrenzung des Körpers (14) vorsteht und über das Nadelende als Membrane eine schlauchartige zentrische Kappe (24) gestülpt ist, die das Nadelende des Nadelteiles (12) fest umschließt, daß der Umfangsrand der übergestülpten Membrane (24) zwischen den Körpern (9, 14) festgelegt ist, und daß die Membrane (24) am äußersten Ende eine elastische Durchtrittsstelle (27) aufweist, die so bemessen ist, daß sie bedingt durch den vom Kolben (20) auf das Insulinvolumen ausgeübten Druck öffnet und Insulin bei der Injektion in das Nadelteil (10) durchfließen läßt, und bei Entlastung des Kolbens (20) durch die Elastizität des Materials der Membrane schließt und den Insulindurchfluß sperrt (Fig. 3).

10. Vorrichtung nach Anspruch 9, dadurch gekennzeichnet, daß die Membran eine Ringwulst (26) aufweist.

11. Vorrichtung nach Anspruch 1, dadurch gekennzeichnet, daß die Durchflußsperrvorrichtung (15) ein Einwegventil (28), z.B. ein Kugelventil ist, im Ruhezustand die Kugel (29) durch die Druckfeder (30) in Richtung Ampulle (2) die Nadel (12) verschließt, das bei Insulindurchfluß durch Kolbenbetätigung sich öffnet und bei Entlastung des Kolbens schließt (Fig. 4).

12. Vorrichtung nach Anspruch 1, dadurch gekennzeichnet, daß die Durchflußsperrvorrichtung (35) eine Folie aus elastisch nachgiebigem Material mit einer im wesentlichen zentrischen Durchflußöffnung (35') ist, daß die einander zugewandten Flächen (31, 32) der beiden Körper (9, 14) die Folie (35) positionierend und festklem-mend aufnehmen, und daß die Fläche (31) eine kontinuierlich umlaufende, ringförmige Erhebung (33) sowie die Fläche (32) eine mit der Erhebung (33) konforme, kontinuierlich umlaufende ringförmige Vertiefung (34) aufweist, bzw. alternativ Erhebungen und Vertiefungen in den Flächen (31, 32) miteinander vertauscht sind (Fig. 5 und Fig. 6).

13. Vorrichtung nach Anspruch 1, dadurch gekennzeichnet, daß die Durchflußsperrvorrichtung (38) eine Folie aus elastisch nachgiebigem Material mit einer im wesentlichen zentrischen Durchtrittsstelle (39) ist, daß die einander zugewandten Flächen (36, 37) der beiden Körper (9, 14) die Folie positionierend und festklemmend aufnehmen, und daß die Flächen (36, 37) im wesentlichen plane Flächen sind, auf deren zumindest einer die Folie (38) festgelegt bzw. festgeklebt sein kann (Fig. 7).

14. Vorrichtung nach Anspruch 1, dadurch gekennzeichnet, daß die Körper (9, 14) eine Ausnehmung (40) bilden, in der die Membrane bzw. Folie eine Ausweichbewegung beidseitig der Folienebene ausführen kann (Fig. 7).

15. Vorrichtung nach Anspruch 12, dadurch gekennzeichnet, daß die einander zugewandten Flächen (36, 37) zur Positionierung der Membran (38) aufgerauht, geriffelt oder mit dergl. Unebenheiten versehen ausgebildet sind.

16. Vorrichtung nach Anspruch 1, dadurch gekennzeichnet, daß der Körper (41) eine Ausnehmung (45) aufweist, in die ein zylindrischer, die Nadel (12) aufnehmender Ansatz (41') des Körpers (41) vorsteht, daß der Ansatz (41') am bzw. in der Nähe des freien Endes eine Ringwulst oder dergleichen flanschartige Erweiterung (42) besitzt, daß eine Membrane (43) in Form einer Kappe auf dem Ansatz (41') aufgesetzt ist, und daß die die Ausnehmung begrenzende Umfangswand (47) in Anlage mit der Membrane (43) steht und sie festlegt.
